(19) Europäisches Patentamt
European Patent Office
Office européen des brevets

(11) Publication number: 0 268 446 A2

# (12) EUROPEAN PATENT APPLICATION

(21) Application number: 87310100.0

(22) Date of filing: 16.11.87

(51) Int. Cl.4: C 12 P 21/00
C 12 N 15/00

(30) Priority: 14.11.86 US 931694

(43) Date of publication of application:
25.05.88 Bulletin 88/21

(84) Designated Contracting States:
AT BE CH DE ES FR GB GR IT LI LU NL SE

(71) Applicant: THE UNIVERSITY OF BRITISH COLUMBIA
No.300-6174 University Boulevard
Vancouver British Columbia (CA)

(72) Inventor: Levy, Julia G.
2034 West 36th Avenue
British Columbia (CA)

Steele, Kevin J.
4525 Juniper Place
Victoria, V8N 3KI (CA)

Stammers, Anthea Tench
1134 Lombardy Drive
Port Coquitlam V3B 5P5 (CA)

(74) Representative: Goldin, Douglas Michael et al
J.A. KEMP & CO. 14, South Square Gray's Inn
London WC1R 5EU (GB)

The microorganism(s) has (have) been deposited with American Type Culture Collection under number(s) HB-9588 and HB-9587.

## (54) An antiidiotypic T-cell hybridoma.

(57) A method for preparing a T cell suppressor factor representing the antiidiotypic second tier of the T suppressor cascade is described. A subject animal is immunized with a primary antigen-specific suppressor factor (TsF1), and the resulting T cells are immortalized and screened for those secreting the desired factor (TsF2). Screening is accomplished by immunoreactivity assay against anti-T suppressor antibody and against TsF1. A particular TsF2, which is specifically antiidiotypic with respect to P815 tumor antigen is disclosed, along with the immortalized A29 cell line which produces it.

EP 0 268 446 A2

## Description

# AN ANTIIDIOTYPIC T-CELL HYBRIDOMA

### Technical Field

The invention relates to the regulation of the immune response to tumors; in particular, it relates to a cell line and the products thereof useful in controlling tumor growth in vertebrate subjects.

### Background Art

After a decade of startling progress, the precise mechanisms involved in an immune response to stimulation by introduction of a foreign antigen is still incompletely understood. Nevertheless, the current model is considerably more detailed than that available only a few years ago. Of relevance to the present invention is the T-cell mediated response to antigens, in particular to tumor antigens. Three basic types of T-cells are believed to be involved: cytotoxic (killer) cells, helper cells, and suppressor cells. In broadest outline, the ultimate demise of the antigen-bearing tumor is believed due to interaction with the cytotoxic T-cells in conjunction with the helpers. This interaction is, however, regulated by a T-suppressor cascade.

The T-suppressor cascade is shown diagrammatically below:

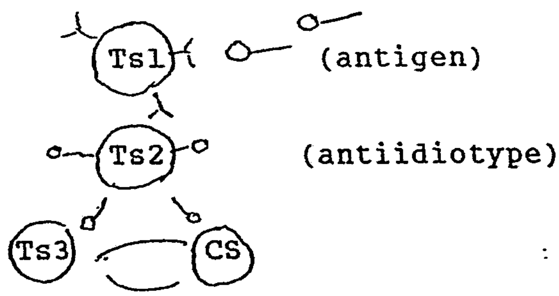


The designations Ts1, Ts2, and Ts3 are those of Sy, M.S., et al, J Exp Med (1981) 153:1415. Briefly, the end products of this cascade, Ts3 (also called effectors) suppress the function of the T helper cells in assisting the cytotoxic lymphocytes (CTL) in destroying the antigen-labeled tumor. Thus, they negate the effective immune response to the tumor. These cells are, however, in competition with "contrasuppressors", designated CS. The formation of these competitive cell types is regulated by antiidiotype factors secreted by the second order Ts2 cells. This secretion is stimulated by a TsF1 idiotypic factor secreted by the initially contacted and first level Ts1 cells, which respond directly to the antigen.

Thus, the Ts1 cells respond to antigens by forming idiotype-positive (id+) factors complementary to a specific epitope on the antigen. These complementary idiotypes generate the antiidiotypic (id−) Ts2 factors which, therefore, resemble the original antigen. These factors affect the performance of the Ts3 and CS cells, which must, then, in turn, be id+.

Certain factors significant in this system are known in the art. (see, e.g., Yamauchi, K., et al, Eur J. Immunol (1983) 13:285; Sorensen, C.M., et al, J Immunol (1985) 135:362; Jensen, P.E., et al, J Immunol (1986) 136:1309; Flood, P.M., et al, J Immunol (1985) 135:933; Hirai, Y., et al, J Immunol (1981) 126:2064; Jayaraman, S., et al, J Immunol (1983) 130:2519; Singhai, R., et al, Eur J Immunol (1985) 15:526.) In particular, the T suppressor immune response to P815, a tumor of DBA/2 mice, has been studied (Takei, F., et al, J Immunol (1977) 118:412; ibid, (1978) 120:1218; Mills C.D., et al, Transplantation (1985) 39:202). The id+ factor secreted by the Ts1 cells in response to the murine tumor P815, has been isolated in the murine system (Steele, J.K., et al, Cellular Immunol (1985) 90:303-313; Steele, J.K., et al, Cellular Immunol (1985) 90:303-313; Steele, J.K., et al, J Immunol (1985) 134:2767-2778) and a similar factor has been found in human tonsil T-cells (Steele J.M., et al, J Immunol (1985) 135:1201-1206). In both human and murine systems, this factor is apparently a protein of molecular weight 70-90 kd and may be a dimer.

It has been possible to screen for cells secreting this factor (TsF1) by identifying them with a monoclonal antibody, B16G. B16G is secreted by immortalized cells from animals immunized with a T-cell population generated upon implantation or injection of P815 tumor. This antibody, however, seems to bind to a constant epitope region of the TsF1, and is not antigen specific (Maier. T,.A., et al, J Immunol (1983) 131:1843). The T-cell factor (TsF1) is immunoreactive with both P815 antigen and with the B16G antibody.

While the TsF1 secreted by Ts1 cells is useful as a vaccine to generate antibodies which combat its suppressive effect on tumors (see copending US Appln. Serial No. 112,974 filed 23rd Oct. 1987). It appears, according to the current model, that the ability to mimic the performance of the Ts2 population is central to the immunoregulatory effect of the cascade. Thus, if Ts2-type cells and their secretion products (TsF2) were available, the activities of the Ts3 and CS cells which form the products of the cascade could be more

intimately regulated.

Disclosure of the Invention

The invention provides cell lines and factors secreted by them that are important in the regulation of immune responses to tumors. The cell lines are produced by immortalizing T-cells generated in response to injection of TsF1 or its equivalent and screened by binding to an antibody that is immunoreactive with both the injected TsF1 and TsF2 factors of the invention. The identified immortalized cells are then cultured in order to obtain desired amounts of the secreted factor.

Accordingly, in one aspect, the invention is directed to cell lines and their secretion products which are generated in response to antigen-specific TsF1 stimulation and which bind to antibody cross-reactive with TsF1 and TsF2.

In another aspect, the invention relates to a method to obtain such cell lines.

Modes of Carrying Out the Invention

The invention herein discloses a general method for obtaining a TsF2 factor which is specifically antiidiotypic in relation to a specific antigen. Hybridomas which secrete the desired factor can be prepared by immortalizing T cells from an appropriately immunized animal.

Thus, in general, preparation of immortalized cell lines which secrete the desired factors utilizes immunization and immortalization techniques known in the art which are adapted to the purpose to be served by the invention. A subject mammal is immunized with the desired, antigen-specific, TsF1 and the spleen or peripheral blood lymphocytes immortalized, for example, according to the procedure of Kohler and Milstein or by infection with virus. The immortalized cells are then screened for reactivity using an appropriate protocol. Cell lines obtained in this way are used as sources for the desired proteinaceous factor, which is secreted when the cells are cultured in vitro or grown as ascites tumors, as is understood in the art.

The general methods, therefore, to prepare hybridomas, or other immortalized cells, in general are known. Critical to obtaining the desired antigen-specific factors of the invention is the proper selection of immunogen and the proper design of a screening procedure for the factor-secreting immortalized cells that are prepared.

The appropriate immunogen is TsF1 factor which is specific for the same antigen as the desired antigen specificity of the TsF2 factors of the invention. The TsF1 factor is prepared in an analogous way to that here described for TsF2, but immunization is with materials bearing the specific antigen and the immortalized cells producing it are screened using the double criterion of binding to an antibody reactive with and specific for T cell suppressor factors in general, and with the antigen to which the TsF1 factor is specifically reactive. One such TsF1 factor is described and utilized below--that immunoreactive with an antigen specific to the P815 tumor in DBA/2 mice. However, alternative TsF1 antigen-specific factors could also be used. Exemplary of such factors are those produced in response to administration of such antigens as those residing on various human tumors, those residing on infective agents, especially viral and bacterial infective agents, and so forth.

Thus, using methods similar to those of Steele, J.K. et al (supra), any antigenic material can be used to stimulate the production of T cells secreting TsF1 factors specific for it (id+). These TsF1 factors can then be produced by immortalizing the appropriate T cells and screening for those which secrete the desired factor. The appropriate criteria for this screen are immunoreactivity with the antigen combined with immunoreactivity for an antibody which is generically reactive with T cell suppressor factors.

Such antibodies are currently available. A particularly useful antibody is B16G, which has been shown to be generically reactive to T suppressor factors, regardless of their antigen specificity. B16G is deposited with ATCC and has deposit no. HB9588.

The availability of antibodies useful for screening immortalized T cell populations for suppressor factors in general eases the production of any desired TsF1 component so as to be immunoreactive with any desired antigen. This TsF1 is useful as an immunogen for the production of the TsF2 factors of the invention.

The other aspect critical for preparing the TsF2 factors of the invention is the choice of an appropriate screening procedure. Since the TsF2 factors are antiidiotypic, they mimic the antigen. In addition they react with antibodies such as B16G, which are generally reactive with T suppressor factors. In view of these two properties, a double criterion for screening is appropriate. The immortalized T cells obtained from animals immunized with the appropriate TsF1 are screened by choosing those which react both with the anti-suppressor factor antibody such as B16G and with an idiotypic antigen-specific factor. This factor would be either the appropriate TsF1 immunogen, or could also be antibodies raised to the original antigen. By using this combination of reactivities, only those T cells secreting the desired TsF2 factors are obtained.

The TsF2 factors of the invention are, in general, of molecular weight approximately 70 kd. They are proteins, and may be glycosylated. Their properties are well defined. They are reactive with antibody generically specific for T cell suppressor factors, but they are antigen-specific. They are antiidiotypes and therefore react with TsF1 specific to the antigen in question and to antibodies raised against that antigen, as well. They do not bind to the antigen per se. These materials suppress the generation of cytotoxic lymphocytes to the specific antigen in in vitro assays and, further, remove factors from spleenocytes which suppress the formation of cytotoxic lymphocytes.

Utility

The TsF2 factors of the invention are useful in regulating the immune system. Because they are central to the suppressor cascade, and because the products of the cascade which they control have contravening effects, they can be used in appropriate circumstances either to enhance or suppress the function of the immune system in response to a specific antigen. In addition, antibodies raised against these factors are capable of complementary immune regulation.

The factors therefore are useful in treating disorders of the immune system and in treating conditions which are responsive to it. For example, autoimmune diseases would be subject to regulation by administering TsF2 specific for the antigen to which the unwanted immune response occurs.

The TsF2 of the invention is prepared using an isolated immortalized cell line. TsF2 is therefore produced in a form which is homogeneous and free of the immune system components which normally accompany it. As used herein, the phrase "substantially free of impurities" refers to TsF2 produced in this way. While it may be accompanied by materials normally produced by the immortalized Ts2 cell, it is free of materials which normally accompany its production in situ.

Examples

The following examples are intended to illustrate but not limit the invention.

Example 1

Preparation of Immunogen

The immunogen used to stimulate production of T-cells capable of secreting the desired TsF2 factor was prepared by affinity purification of the P815 antigen-specific factor TsF1. The hybridoma cell line, A10, known to produce murine TsF1 specific for P815 antigens (Steel, J.K., et al, J Immunol (1985) 134:2767-2778 (supra)) was cultured as an ascites tumor in CBAxDBA/2 F1 mice which had received 0.5 ml of pristane 7 days earlier and 500 rad on the day of injection. Approximately $10^6$ cells were injected intraperitoneally. The ascites thus obtained was diluted 5 × in PBS, and cells were removed by centrifugation. The ascites fluid was then affinity purified using either B16G antibodies or P815 membrane extracts linked to Sepharose 4B, as described by Cautrecasas, P., J Biol Chem (1970) 245:3059. The columns containing the adsorbed TsF1 were washed with PBS and the adsorbed material was eluted with ice-cold 0.1 N HCl. The elution pattern was monitored (after immediate neutralization) at 280 nm. The protein-containing fractions were pooled, dialyzed against PBS, and used within 24 hours. All procedures were conducted at 4°C.

Example 2

Immunization and Production of Hybridoma

The putative TsF1 prepared in Example 1 was injected IP into DBA/2 mice, 20 mg/animal, and after 6 days the animals were sacrificed and thymus and spleen tissues cultured for fusion with BW5147 cells using a polyethylene glycol fusion procedure and selection in HAT medium (Steele, J.K., et al, J Immunol (1985), (supra)). The immortalized hybridomas were plated into microtiter wells and screened, after obvious growth of clones was visible, by testing the supernatant with a sandwich ELISA for binding to both the A10 cell-secreted factor and to B16G antibody.

For the assay, ELISA Immulon-II plates were coated with the affinity-purified immunogen of Example 1 at a concentration of 50 µg/ml. The culture supernatants to be tested were then added to the plates, allowed to bind for 2 hours, washed, and then developed with B16G.

Surprisingly, rather than blocking the ability of B16G to bind to the TsF1 coating on the plates, the TsF2-containing supernatants, which were bound to the plates by TsF1 were capable of reacting with B16G and thus enhanced B16G binding. The B16G antibody is thus generic to suppressor factors. Of over 2,500 hybrids screened, only 1 gave positive results in this assay. This hybridoma, designated A29, was cloned three times initially and has been maintained in vitro in DME plus 20% FCS with a continuous feeder layer of DBA/2 spleenocytes. The hybrid is not capable of independent growth and required either a dense feeder layer or conditioned medium containing IL-2. A29 was deposited at ATCC on 13 November 1987 under terms of the Budapest Treaty, and has Accession No. HB9587.

A29 can be maintained either in tissue culture or grown as ascites in pristane-treated, irradiated F1 animals.

Example 3

Purification of TsF2 from A29

The soluble, proteinaceous product of A29 is purified from culture supernatants or ascites by affinity purification on B16G-Sepharose or A10-Sepharose columns. Only 1-2 µg of TsF2/1 per liter are produced from cultures of the A29 cells. SDS-polyacrylamide gel electrophoresis of the purified fractions using 10% gels and staining with Coomassie blue shows TsF2 to have a molecular weight of approximately 70 kd.

Example 4

The Effect of TsF2 on Tumor Growth

The affinity-purified TsF2 of Example 3 was injected IV into groups of DBA/2 mice at 100 μg/mouse simultaneously with subcutaneous inoculation with $10^4$ P815 cells in the right flank. Controls received either PBS or an irrelevant factor at equal concentrations. The mice were monitored daily to assess tumor growth. These studies gave mixed results, but, generally, enhanced tumor growth resulted.

Example 5

Effect of TsF2 In Vitro on Generation of Cytotoxic Lymphocytes Specific for P815

In general, to conduct the assay, DBA/2 spleen cells were plated in doubling dilutions from suspensions of $10^6$ to $1.25 \times 10^5$ cells into V-bottom plates at 100 μl aliquots per well in replicates of 8. P815 cells from ascites fluid of DBA/2 mice, washed three times, incubated at $2\text{-}6 \times 10^6$ cells/ml with 50 μg/ml mitomycin C in complete RPMI medium at 37° C and 5% $CO_2$ for 1 hour, were harvested and washed and resuspended in complete RPMI. 100 μl of the resulting suspension containing $2.5 \times 10^4$ cells was added to each well containing the spleen cells. Five days later, the cell mixtures were resuspended, and 100 μl from each well was transferred to U-bottom plates.

Fresh P815 cells were labeled for 1-1/2 hours with chromium-51 at 0.2 mCi per $2 \times 10^6$ cells. The cells were washed and incubated at 37° C in 5% $CO_2$ in complete RPMI for 3-4 hours. The labeled cells were washed 3 times, resuspended in complete RPMI, and 100 μl aliquots containing $10^4$ cells were added to all wells, including 2 rows of 8 replicates of labeled P815 alone as a control. The plates were centrifuged at 100 rpm for 5 minutes and then incubated at 37° C, 5% $CO_2$, for 18 hours.

The plates were then again centrifuged, and 100 μl supernatant was removed from all wells (except those containing P815 cells along) to calculate chromium release. For wells containing P815 cells along, 100 μl cell suspension was removed to calculate maximum chromium CPM which could have been released had all the P815 cells been killed. Samples were counted in a Picker Pace 1 γ counter, and percent specific lysis was calculated as the ratio of

$$\frac{\text{Sample CPM - spontaneous CPM}}{\text{Maximum CPM - spontaneous CPM}} \times 100$$

To test the effects of the factors secreted by the T suppressor cascade, affinity-purified TsF2 or control extracts from BW5147 were added at 1, 0.5, and 0.1 μg/ml to the wells along with the chromium-labeled cells. The A29-secreted TsF2 significantly suppressed the formation of cytotoxic lymphocytes. At all concentrations, the % lysis was substantially lower for TsF2-treated wells than for controls.

In other experiments, A29-secreted TsF2 was shown to lack a generally immunosuppressive effect; thus, the effect on CTLs is antigen-specific.

The result above was further confirmed by conducting the foregoing assay but as modified by panning the DBA/2 spleenocytes prior to plating them by passing them over plates containing 16 μg/ml of affinity-purified TsF2 (or control material from BW5147). In the panning, the plastic petri plates were coated overnight with the purified TsF2 in 5 ml PBS, the plates washed 3 times with PBS, blocked for 1 hour at 4° C with 10% FCS in PBS, and then washed with PBS again. The DBA/2 spleen cells were washed, resuspended in DME plus 5% FCS, added to the plates at $5 \times 10^7$ cells per plate, and incubated for 1 hour at 4° C. The nonadherent, panned cells were thus removed, counted, and used in the CTL assay as described above.

The results showed that when spleen cells were first panned against TsF2, they had enhanced ability to effect lysis in the assay, indicating that the TsF2 panning removed a component of the spleen cell population which suppresses CTL.

Example 6

Responsiveness of A29 Cells to A10 and B16G

Changes in the level of calcium ion within a cell is a measure of its specific membrane interaction, presumably through receptors, with materials binding to these receptors. The intracellular calcium ion concentration can be determined by the method of Pozzan, T., et al, J Cell Biol (1982) 94:335-340. The hybridoma of the invention, A29, was subjected to this assay in the presence of TsF1 purified from A10 cells or in the presence of B16G antibody. Both were shown to cause significant changes at the membrane of A29.

In the converse experiment, TsF2 purified from A29 was shown to cause significant calcium flux of A10 cells (as does B16G).

Therefore, the equivalent stimulation between id+ and id- cell populations supports the network concept illustrated.

## Claims

1. A composition of matter which consists essentially of T cell suppressor factor secreted by Ts2 cells (TsF2) substantially free of impurities.
2. The TsF2 of claim 1 which is a protein of 70 kd.
3. The TsF2 of claim 1 or 2 which is immunoreactive with B16G.
4. The TsF2 of any one of the preceding claims which is produced by an hybridoma prepared from T cells of a mammal immunized with TsF1 specific for a tumor antigen.
5. An immortalized cell line capable of secreting the factor of any one of the preceding claims.
6. The cell line of claim 5 which is A29.
7. A method to produce cells which secrete TsF2 having specificity for a desired antigen, which method comprises
   (a) immunizing an animal with a TsF1 factor specific for said antigen;
   (b) obtaining T cells from the immunized animal;
   (c) immortalizing said T cells; and
   (d) screening the immortalized T cells for production of said TsF2 by selecting those cells which are immunoreactive with antigen generally reactive with suppressor factors and which are reactive with the TsF1 of (a).
8. A method to produce TsF2 having specificity for a desired antigen, which method comprises culturing the cells of claim 5 or 6.
9. A method to produce TsF2 having specificity for a desired antigen, which method comprises culturing the cells produced by the process of claim 7.
10. A method to regulate the immune response of a subject vertebrate to a specific antigen which comprises administering to said subject an effective amount of the TsF2 of any one of claims 1-4.